(19) Europäisches Patentamt
European Patent Office
Office européen des brevets

(11) EP 1 907 067 B2

(12) # NEW EUROPEAN PATENT SPECIFICATION
After opposition procedure

(45) Date of publication and mention of the opposition decision:
**28.06.2017 Bulletin 2017/26**

(45) Mention of the grant of the patent:
**04.09.2013 Bulletin 2013/36**

(21) Application number: **06788450.2**

(22) Date of filing: **26.07.2006**

(51) Int Cl.:
***C08F 290/06*** *(2006.01)*

(86) International application number:
**PCT/US2006/028870**

(87) International publication number:
**WO 2007/016105 (08.02.2007 Gazette 2007/06)**

(54) **POLYMERS WITH PENDANT POLY(ALKYLENEOXY) SUBSTITUENT GROUPS AND THEIR USE IN PERSONAL CARE APPLICATIONS**

POLYMERE MIT HÄNGENDEN POLY(ALKYLENOXY)-SUBSTITUENTENGRUPPEN UND IHRE VERWENDUNG IN KÖRPERPFLEGE-ANWENDUNGEN

POLYMERES AYANT DES GROUPES SUBSTITUANTS DE POLY(ALKYLENEOXY) PENDANTS ET LEUR UTILISATION DANS DES APPLICATIONS DE SOINS D'HYGIENE PERSONNELLE

(84) Designated Contracting States:
**AT BE BG CH CY CZ DE DK EE ES FI FR GB GR HU IE IS IT LI LT LU LV MC NL PL PT RO SE SI SK TR**

(30) Priority: **26.07.2005 US 702413 P**

(43) Date of publication of application:
**09.04.2008 Bulletin 2008/15**

(73) Proprietor: **Solvay USA Inc.**
**Cranbury, NJ 08512 (US)**

(72) Inventors:
- **CRISTOBAL-LUMBROSO, Gabriella**
  **E-28017 Madrid (ES)**
- **LIZARRAGA, Gilda**
  **Cranbury, NJ 08512 (US)**

(74) Representative: **Busher, Samantha Jane et al**
**Elkington and Fife LLP**
**3-4 Holborn Circus**
**London, EC1N 2HA (GB)**

(56) References cited:
**JP-A- H07 206 627**
**US-A1- 2004 137 047**
**US-A1- 2005 152 855**
**US-A1- 2005 159 353**

- **CARENZA, MARIO ET AL: "Hydrogels obtained by radiation-induced polymerization for yeast cells immobilization" EUROPEAN POLYMER JOURNAL , 29(7), 1013-18 CODEN: EUPJAG; ISSN: 0014-3057, 1993, XP002566985**
- **'CARBOWAX PEGs for Industrial Applications' DOW, [Online] 06 December 2004, XP003014302 Retrieved from the Internet: <URL:http://www.dow.com/polyglycols/carbowax/products/peg.htm>**

EP 1 907 067 B2

## Description

Field of the Invention

This invention relates to polymers, more particularly to certain polymers with pendant poly(alkyleneoxy) substituent groups and the use of such polymers in personal care applications.

Background of the Invention

Products designed solely for cleaning hair can leave the hair tangled and unmanageable. Conditioning polymers and benefit agents for improving hair condition by reducing combing friction and by providing a soft smooth feel and shiny appearance to the hair are known. However, in many cases such polymers and benefit agents may also produce some undesirable effects, such as a decrease in hair volume due to buildup of the polymer conditioner on the hair and a detrimental effect on hair body due to a decrease in friction between the hair fibers.

There is an interest in developing new ways to improve volume properties of the hair.

Summay of the Invention

In a first aspect, the present invention is directed to a polymer comprising, based on the total number of monomeric units of the polymer, greater than 50 percent monomeric units that comprise, per monomeric unit, at least one pendant poly(alkyleneoxy) substituent group according to formula (I):

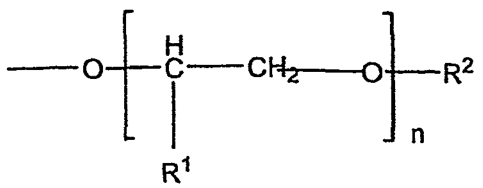

(I)

wherein:

each $R^1$ is independently H, methyl or ethyl,
each $R^2$ is independently H or $(C_1-C_6)$alkyl,
each n is independently an integer of from about 5 to about 500, and exhibiting a weight average molecular weight of from about 1,000 to about 1,000,000 grams per mole.

and wherein each of the monomeric units that comprise at least one pendant poly(alkyleneoxy) substituent group according to formula (I) is derived from a monomer according to formula (II):

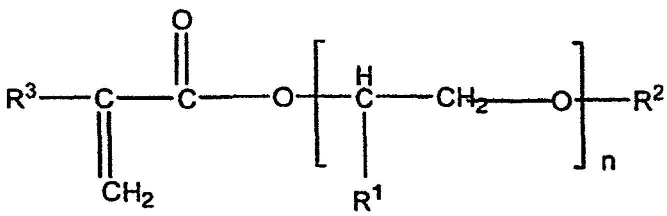

(II)

wherein:

each $R^1$ is independently H, methyl or ethyl,
each $R^2$ is independently H or $(C_1-C_6)$alkyl,
each $R^3$ is independently H or $(C_1-C_4)$alkyl, and
each n is independently an integer of from about 5 to about 500.

In a second aspect, the present invention directed to a personal care composition comprising the above-described polymer and a cosmetically acceptable carrier.

In a third aspect, the present invention is directed to a method, for treating hair or skin, comprising contacting the hair or skin with the above-described personal care composition.

The polymer of the present invention imparts volume properties to the hair.

Detailed Description of Invention and Preferred Embodiments

The term "pendant", as used herein in reference to a poly(alkyleneoxy) substituent group means that such group is present as a side group that is attached to a polymer chain.

As used herein, the term "alkyl" means a saturated hydrocarbon radical hydrocarbon radical, which may be straight or branched, such as, for example, methyl, ethyl, n-propyl, iso-propyl, n-butyl, sec-butyl, t-butyl, pentyl, n-hexyl,

As used herein, the terminology "(Cp-Cq)" in reference to an organic group, wherein p and q are each integers, indicates that the group may contain from p carbon atoms to q carbon atoms per group.

As used herein, the terminology "ethylenic unsaturation" means a terminal (that is, a, β) carbon-carbon double bond.

As used herein, an indication certain substituent group of an embodiment of the invention is "as described above" refers separately to each previous description, including the broadest description and any narrower descriptions, of such substituent group that is applicable in the context of that embodiment.

In one embodiment, the polymer comprises, based on the total number of monomeric units of the polymer, greater than or equal to about 80 percent (%), more typically greater than or equal to about 95%, and even more typically greater than or equal to about 99%, monomeric units that comprise, per monomeric unit, at least one, more typically one, pendant poly(alkyleneoxy) substituent group according to formula (I), wherein $R^1$, $R^2$, and n are each described as above, and wherein each of the monomeric units that comprise at least one pendant poly(alkyleneoxy) substituent group according to formula (I) is derived from a monomer according to formula (II), wherein $R^1$, $R^2$, $R^3$ and n are each described as above.

In one embodiment, each of the monomeric units of the polymer comprises at least one, more typically one, pendant poly(alkyleneoxy) substituent group according to formula (I) wherein each $R^1$ and each $R^2$ is independently H or $CH_3$, and each n is independently an integer of from about 5 to about 100, more typically, from about 5 to about 50, even more typically from about 5 to about 25, and wherein each of the monomeric units that comprise at least one pendant poly(alkyleneoxy) substituent group according to formula (I) is derived from a monomer according to formula (II), wherein $R^1$, $R^2$, $R^3$ and n are each described as above.

In one embodiment, each of the monomeric units of the polymer comprises at least one, more typically one, pendant poly(alkyleneoxy) substituent group according to formula (I), wherein $R^1$, $R^2$, and n are each described as above, and wherein each of the monomeric units that comprise at least one pendant poly(alkyleneoxy) substituent group according to formula (I) is derived from a monomer according to formula (II), wherein $R^1$, $R^2$, $R^3$ and n are each described as above.

In one embodiment, the polymer is a homopolymer wherein all of the monomeric units of the polymer are identical and each monomeric unit comprises at least one, more typically one, pendant poly(alkyleneoxy) substituent group according to formula (I), wherein $R^1$, $R^2$, and n are each described as above, and wherein all of the monomeric units that comprise at least one pendant poly(alkyleneoxy) substituent group according to formula (I) is derived from a monomer according to formula (II), wherein $R^1$, $R^2$, $R^3$ and n are each described as above.

In one embodiment, each of the monomeric units that comprise, per monomeric unit, at least one pendant poly (alkyleneoxy) substituent group according to formula (I) is derived from a monomer according to formula (II):

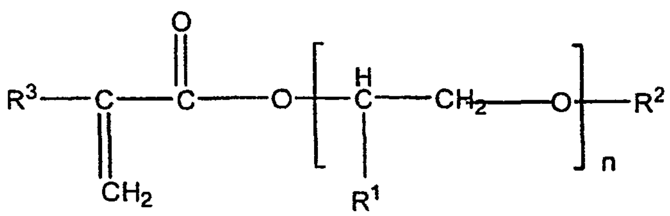

(II)

and each of such monomeric units is according to formula (III):

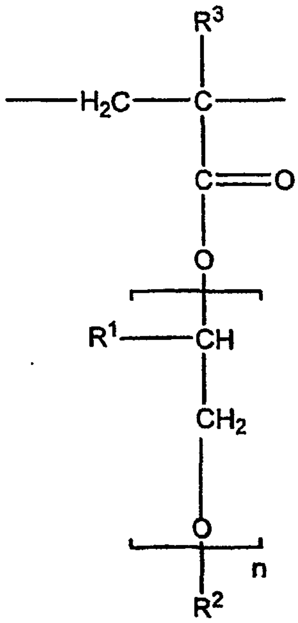

(III)

wherein:

each $R^3$ is independently H or $(C_1-C_4)$alkyl,
$R^1$, $R^2$, and n are each defined as above.

Suitable monomers according to formula (II) include, for example, ethoxylated hydroxyethyl acrylate, ethoxylated hydroxyethyl methacrylate, propoxylated hydroxyethyl acrylate, propoxylated hydroxyethyl methacrylate, butoxylated hydroxyethyl acrylate, and butoxylated hydroxyethyl acrylate.
In one embodiment of the polymer comprising monomeric units according to structure (III), each $R^1$, each $R^2$, and each $R^3$ is independently H or $CH_3$, and each n is independently an integer of from about 5 to about 100, more typically, from about 5 to about 50, even more typically from about 5 to about 25.
In one embodiment, all monomeric units of the polymer are according to structure (III), wherein $R^1$, $R^2$, $R^3$, and n are each as described above.
In one embodiment, the polymer of the present invention is a homopolymer wherein all monomeric units of the polymer are identical monomeric units according to structure (III), wherein $R^1$, $R^2$, $R^3$, and n are each as described above.
In one embodiment, the polymer further comprises monomeric units that do not comprise any poly(alkyleneoxy) substituent groups according to formula (I). Such units may be derived, for example, by copolymerizing one or more ethylenically unsaturated monomers that do not comprise any poly(alkyleneoxy) substituent groups according to formula (I) with one or more ethylenically unsaturated monomers that contain poly(alkyleneoxy) substituent groups according to formula (I), wherein $R^1$, $R^2$, and n are each described as above, and wherein all of the monomeric units that comprise at least one pendant poly(alkyleneoxy) substituent group according to formula (I) is derived from a monomer according to formula (II), wherein $R^1$, $R^2$, $R^3$ and n are each described as above.
In one embodiment, the polymer of the present invention comprises, based on the total number of monomeric units of the polymer:

(a) from about 90 % to less than 100 %, more typically from about 95 % to less than 100 %, even more typically from about 99 % to less than 100 %, first monomeric units each of which:

(i) comprises, per monomeric unit, at least one pendant poly(alkyleneoxy) substituent group according to formula (I), wherein $R^1$, $R^2$ and n are each as described above, and wherein all of the monomeric units that comprise at least one pendant poly(alkyleneoxy) substituent group according to formula (I) is derived from a monomer according to formula (II), wherein $R^1$, $R^2$, $R^3$ and n are each described as above and/or
(ii) is according to structure (III), wherein $R^1$, $R^2$, $R^3$, and n are each as described above, and

(b) from greater than 0 % to about 10 %, more typically from greater than 0 % to about 5 %, even more typically from greater than 0 % to about 1 %, second monomeric units that do not comprise any poly(alkyleneoxy) substituent groups according to formula (I).

Suitable ethylenically unsaturated comonomers that do not comprise any poly(alkyleneoxy) substituent groups according to formula (I) are known compounds and include, for example, esters of ethylenically unsaturated carboxylic acids, such as ethyl acrylate, methyl methacrylate, n-propyl acrylate, n-propyl methacrylate, n-butyl acrylate, n-butyl methacrylate, 2-ethylhexyl acrylate, 2-ethylhexyl methacrylate, hydroxyethyl acrylate, hydroxyethyl methacrylate, hydroxypropylacrylate, hydroxypropylmethacrylate, cyclohexyl acrylate, cyclohexyl methacrylate, N,N-dimethylaminoethyl acrylate methyl chloride, N,N-dimethylaminoethyl methacrylate methyl chloride, benzyldimethylammonium ethyl acrylate chloride, and benzyldimethylammonium ethyl methacrylate chloride, amides of ethylenically unsaturated carboxylic acids, such as acrylamide, methacrylamide, trimethylammonium propyl acrylamide chloride, and trimethylammonium propyl methacrylamide chloride, vinyl monomers, such as vinyl acetate, vinyl versatate, vinyl pyrrolidinone, and vinyl caprolactam, and allylic monomers, such as allyl alcohol.

In one embodiment, the polymer of the present invention exhibits a weight average molecular weight ("Mw") of from about 2,500 to about 900,000 grams per mole ("g/mol"), more typically, from about 10,000 to about 750,000 g/mol.

In one embodiment, the polymer of the present invention exhibits a number average molecular weight ("Mn") of from about 2,500 to about 750,000 g/mol, more typically, from about 10,000 to about 500,000 g/mol.

The Mw and Mn of a polymer are each typically determined by fractionating a solution of the polymer using, for example, size exclusion chromatography, and then determining the molecular weight of each of such polymer fractions, for example, by measuring the intensity of light scattering by the fractions or by measuring the refractive index of the fractions and comparing the refractive index results to those obtained for a polymer of known molecular weight.

In one embodiment, the polymer of the present invention exhibits a polydispersity index, wherein the polydispersity index of the polymer is calculated as the quotient of the Mw of the polymer divided by the Mn of the polymer, of from about 1.2 to about 10, more typically from about 1.4 to about 5.

Methods for making suitable polymers are known in the art. In one embodiment, the polymer according to the present invention is made by known free radical polymerization processes using ethylenically unsaturated monomers (such as, for example, monomers according to formula (II)) that contain poly(alkyleneoxy) substituent groups according to formula (I). In another embodiment, the polymer according to the present invention is made by graft polymerization wherein poly(alkyleneoxy) substituent groups according to formula (I) are added to a polymer backbone having one or more reactive sites at which suitable poly(alkyleneoxy) substituent groups can be added by reaction subsequent to polymerization. Suitable polymer backbones are made by know techniques, such as by polymerizing multi-functional monomers, such as, for example, maleic anhydride, glycidyl methacrylate, vinyl benzyl chloride, hydroxyethyl methacrylate, glycerol monomethacrylate, or a silane monomer such as triethoxyvinyl silane,

In another embodiment, the polymer is made by known controlled free radical polymerization techniques, such as reversible addition fragmentation transfer (RAFT), macromolecular design via interchange of xanthates (MADIX).

As used herein, terminology "personal care composition" means a composition for use in caring for the hair or skin, including, for example, shampoos, hair conditioners, hair gels, hair mousses, hair sprays, skin cleansers, and skin lotions.

As used herein, the term "cosmetically acceptable carrier" means a vehicle that is capable of being mixed with the polymer for topical delivery to the hair or skin and that will not cause harm when applied to the hair or skin. Suitable carriers include water, organic solvents, mixtures of such organic solvents, and mixtures of water and water miscible organic solvents. Suitable organic solvents include, for example, acetone, ($C_1$-$C_{18}$)alkanols, and ($C_7$-$C_{10}$)isoparaffins. Suitable alkanols include, for example, ethanol, isopropanol, Cetyl Alcohol, Stearyl Alcohol, Cetearyl Alcohol, Benzyl Alcohol, Oleyl Alcohol, and mixtures thereof. Suitable water miscible organic solvents include, for example, ($C_1$-$C_4$) alkanols, such as ethanol and isopropanol.

In one embodiment, the personal care composition is a hair styling composition that comprises, based on 100 parts by weight ("pbw") of the composition, from about 0.1 to about 10 pbw, more typically from about 0.1 to about 5 pbw, of a polymer according to the present invention, and from about 90 to about 99.9 pbw of a carrier comprising water.

In one embodiment, the personal care composition is a cleansing composition that comprises a polymer according to the present invention, an aqueous carrier, and one or more surfactants. Suitable surfactants include amphoteric surfactants, Zwitterionic surfactants, nonionic surfactants, anionic surfactants, cationic surfactants, and combinations thereofall of which are well known in the art.

Personal care compositions according to the present invention may, optionally, contain other ingredients, such as, for example, preservatives such as benzyl alcohol, methyl paraben, propyl paraben and imidazolidinyl urea, thickeners and viscosity modifiers such as block polymers of ethylene oxide and propylene oxide, electrolytes, such as sodium chloride, sodium sulfate, polyvinyl alcohol, and ethyl alcohol, pH adjusting agents such as citric acid, succinic acid, phosphoric acid, sodium hydroxide, sodium carbonate, perfumes, dyes, conditioning agents such as organosilicon materials, including, silicone gums, polyorganosiloxane fluids, and silicone resins, i.e., crosslinked polyorganosiloxane systems, propellants, such as propane, butane, isobutane, nitrogen, and carbon dioxide, active ingredients such as antidandruff agents (zinc pyrithion), vitamins or their derivatives such as Vitamin B, Vitamin E acetate, and sequestering agents such as disodium ethylenediamine tetra-acetate. In general, personal care compositions may optionally comprise,

based on 100 pbw of the personal care composition and independently for each such ingredient, up to about 10 pbw, preferably from 0.5 pbw to about 5.0 pbw, of such other ingredients, depending on the desired properties of the personal care composition.

Example 1

The poly(nonaethylene glycol methyl ether acrylate) ("p(nEGA)") homopolymer of Example 1 was made by MADIX and had a theoretical molecular weight of 30,000 g/mol. The p(nEGA) polymer was synthesized by polymerizing 20% by weight nonaethylene glycol methyl ether acrylate monomer (120 g) in a mixture of 4.4:1, water to ethanol (by weight) at 70°C over 17 hours in the presence of an initiator (0.1826 g ammonium persulfate, added as a 25.1 % solution in water plus 2g of rinse water) and a control transfer agent (0.833 g xanthate, Rhodixan A1, Robinson Bros.) with a molar ratio of xanthate to initiator of 5. The monomer was charged to the reactor at the beginning of the reaction and initiator was added batch within five minutes of having made the xanthate addition. The caustic-hydrolyzed polymer exhibited a Mn of 8,600 g/mol and an Mw of 11,800 g/mol, each as determined by size exclusion chromatography and comparison of the refractive index of the eluted fractions with poly(acrylic acid) standards. The molecular weights measured for the hydrolyzed polymer were back-calculated to give a Mn of about 50,000 g/mol and a Mw of about 74,000 g/mol for the original (non-hydrolyzed) p(nEGA) polymer, and the polydispersity index for the non-hydrolyzed polymer was calculated as about 1.5.

An aqueous composition comprising 1.0 wt% of the polymer of Example 1 in water was made by mixing the polymer with water. The effect of such compositions on bulk hair volume was evaluated according to two methods, that is, an image analyzer method which compares the appearance of pre- and post treatment hair tresses, and a ring compressibility method which measures the force or work required to pull a hair tress through a poly(tetrafluoroethylene) O-ring. The methods are each described below.

Pre-and post-treatment tresses pictures (two images per tress imaged at 90° to each other) were capturing by a digital camera and the images were analyzed by digital image analyzer. The volume was determined by measuring the area (corresponding to the surface of the front of the tress' picture) and the depth (corresponding to the thickness of the side tress' picture). The change in volume was determined by the difference between the values of the tresses' volume after and before treatment.

Ten grams of hot melt bound dark brown hair tresses (International Hair Importers) were used. A total of 6 tresses were used for each treatment group. An untreated set of tresses was included as an untreated control group.

The tresses were soaked for 10 minutes in a 15 % by weight aqueous solution of Sodium Lauryl Sulfate (SLS) at a pH of 7.5 to remove residual cationics from the untreated hair. The hair was then stroked 30 times to insure that all the hair fibers have been thoroughly treated with SLS and rinse for 45 minutes under 35-40°C tap water. The tresses were then left to air dry overnight at room temperature.

Each tress was combed for 10 times and shot with a Zerostat™ piezoelectric antistatic pistol to neutralize the static repulsion generated by combing the hair. Hair tresses were measured by simply hanging the tress hair and capturing the images. The images are then analyzed by the Image-Pro digital image analyzer and the untreated tress volume determined for each tress. The tresses are then balanced for volume into sets of 6 each.

The tresses were rinsed under 35-40°C tap water for 1 minute. 1 ml of treatment polymer solution was applied onto wetted tress and massaged for 1 minute. The tresses were then rinsed for 1 minute under 35-40°C tap water. The tresses were then left to air dry overnight at room temperature.

Each tress was combed for 10 times and shot with a Zerostat pistol to neutralize the static repulsion generated by combing the hair. A set of digital post-treatment images were taken and analyzed. The change in volume due to the polymer treatment was then determined.

The parameter "Delta $_{volume}$" was calculated according to equation (A):

$$\text{Delta}_{\text{volume}} = \text{Volume}_{\text{treated}} - \text{Volume}_{\text{untreated}} \qquad \text{(A)}$$

wherein "Volume $_{untreated}$" means the volume measured before treatment with the polymer and "Volume $_{treated}$" means the volume measured after treatment with the polymer. The Delta $_{volume}$ - mean was calculated as the arithmetic average of the Delta $_{volume}$ values for a given treatment condition. In each case, the reported "Lower Limit" and "Upper Limit" are the respective lower and upper limits of a 95% confidence interval for the Delta $_{volume}$ value determined for a given treatment condition.

The data was statistically analyzed using A One Way Analysis of Variance (ANOVA). In each case, the reported "Lower Limit" and "Upper Limit" define an interval around each mean are based on Fisher's least significant different (LSD) procedure at 95% confidence level, i.e., if the mean intervals overlap, then there is no significant difference at

95% confidence between the corresponding treatments.

The % Volume Change was calculated according to equation (B):

$$\% \text{ Volume Change} = \frac{\text{Delta}_{\text{volume}}}{\text{Volume}_{\text{untreated}}} \times 100 \qquad \text{(B)}$$

wherein "Delta $_{volume}$" and "Volume $_{untreated}$" are each defined as above.

The results of the image analysis are set forth in TABLE I below (wherein, and, in accord with the equation (A) above, a positive value of parameter "Delta $_{volume}$" indicates an increase in hair volume after treatment and, in accord with the equation (B) above, a positive value of the parameter "% Volume Change" indicates an increase in hair volume after treatment).

TABLE I

| | Delta $_{volume}$ - mean ($cm^3$) | Lower Limit | Upper Limit | % Volume Change |
|---|---|---|---|---|
| p(nEGA) homopolymer of Example 1 (1% aqueous solution) | 51 | -6 | 109 | 8 |
| Water | -104 | - 161 | -45 | -13 |

Tresses were prepared for ring compression testing in the same manner as described above for the image analysis testing. The parameter "work" necessary to pull each of the hair tresses through a poly(tetrafluoroethylene) O-ring (having an internal diameter of about 18 millimeters and an external diameter of about 25 millimeters (McMaster Carr Supply Part No. 210TEF)) was then measured using the Diastron Tensile tester apparatus. Six measurements per tress were run. The tresses were then balanced for total work into sets of six each

The tresses were then treated with polymer solution in the same manner as described above for the image analysis testing. The parameter "work" necessary to pull each of the treated hair tresses through the internal opening of the poly(tetrafluoroethylene) O-ring was then measured using the Diastron Tensile tester apparatus.

The parameter "delta work" was then determined for each tress according to equation (C):

$$\text{Delta}_{\text{work}} = \text{Work}_{\text{treated}} - \text{Work}_{\text{untreated}} \qquad \text{(C)}$$

where "work $_{treated}$" corresponds to the work measured after treatment with the polymer and "work $_{untreated}$" corresponds to the work measured before treatment with the polymer. The data was statistically analyzed using ANOVA. The intervals around each mean were based on Fisher's least significant different (LSD) procedure at 95% confidence level, that is, if means intervals overlap then there is no significant difference at 95% confidence between the correspondent treatments.

The parameter "percentage of work reduction" is determined for each tress according to equation (D):

$$\% \text{ Work Reduction} = \frac{\text{Delta}_{\text{work}}}{\text{Work}_{\text{untreated}}} \times 100 \qquad \text{(D)}$$

wherein "Delta $_{work}$" and "Work $_{untreated}$". are each as defined above.

The results of the ring compressibility testing are given in TABLE II below (wherein, in accord with equation (C) above, a positive value of the parameter "$\text{Delta}_{work}$ - mean" indicates an increase in hair volume after treatment and, in accord with equation (D) above, a positive value of the parameter "% Work Reduction" indicates an increase in hair volume after treatment).

TABLE II

| | $Delta_{work}$-mean | Lower Limit | Upper Limit | % Work Reduction |
|---|---|---|---|---|
| p(nEGA) homopolymer of Example 1 (1% aqueous solution) | 0.0005 | 0.0003 | 0.0007 | 15 |
| Water | - 0.0008 | - 0.001 | - 0.0006 | -18 |

EXAMPLE 2

The p(nEGA) homopolymer of Example 2 was made by conventional free radical polymerization. p(nEGA) was synthesized by polymerizing 30% by weight NEGA monomer (200g) in water at 75°C over 9 hours in the presence of an initiator (0.0734g of sodium persulfate, added as a 1% solution in water), with a molar ratio of initiator to monomer of 0.07%. 10% of the monomer was fed over 90 minutes while the initiator was added in 5 shots over 80 minutes. The monomer conversion was evaluated by measuring residual acrylic acid, after caustic hydrolysis of the initial p(nEGA) homopolymer: The experimental final concentration measured to be 29.62%. The molecular weight of the polymer (not subjected to caustic hydrolysis) was determined by fractionating a solution of the polymer by size exclusion chromatography and measurement of the intensity of light scattered by the eluted fractions. The polymer exhibited a Mn of 240,000 g/mol, a Mw of 430,000 g/mol, and a polydispersity index of about 1.8.

**Claims**

1. A polymer comprising, based on the total number of monomeric units of the polymer, greater than 50 percent monomeric units that comprise, per monomeric unit, at least one pendant poly(alkyleneoxy) substituent group according to formula (I):

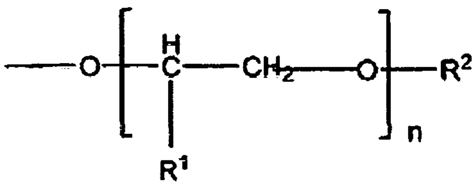

(I)

wherein:

each $R^1$ is independently H, methyl, or ethyl,
each $R^2$ is independently H or $(C_1-C_6)$alkyl, and
each n is independently an integer of from about 5 to about 500, and exhibiting a weight average molecular weight of from about 1,000 to about 1,000,000 grams per mole,

and wherein each of the monomeric units that comprise at least one pendant poly(alkyleneoxy) substituent group according to formula (I) is derived from a monomer according to formula (II):

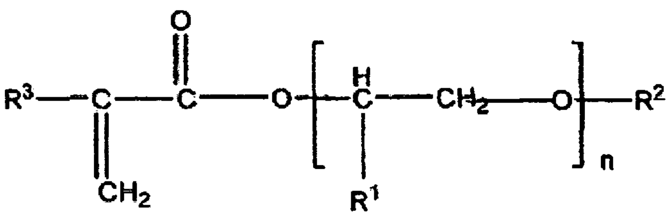

(II)

wherein:

each $R^1$ is independently H, methyl, or ethyl,

each $R^2$ is independently H or $(C_1-C_6)$alkyl,
each $R^3$ is independently H or $(C_1-C_4)$alkyl, and
each n is independently an integer of from about 5 to about 500.

**2.** The polymer of claim 1, wherein the polymer comprises, based on the total number of monomeric units of the polymer, greater than or equal to about 80 percent monomeric units that comprise, per monomeric unit, at least one pendant poly(alkyleneoxy) substituent group according to formula (I).

**3.** The polymer of claim 1, wherein the polymer is a homopolymer.

**4.** The polymer of claim 1, wherein the polymer comprises, based on the total number of monomeric units of the polymer:

(a) from about 90% to less than 100% first monomeric units each of which comprises, per monomeric unit, at least one pendant poly(alkyleneoxy) substituent group according to formula (I), and
(b) from greater than 0% to about 10 second monomeric units that do not comprise any poly(alkyleneoxy) substituent groups according to formula (I).

**5.** A polymer according to claim 1, wherein the polymer exhibits a weight average molecular weight of from about 2,500 to about 900,000 grams per mole.

**6.** A polymer according to claim 1, wherein the polymer exhibits a number average molecular weight of from about 2,500 to about 750,000 grams per mole.

**7.** The polymer of claim 1, wherein each of the monomeric units of the polymer comprises at least one pendant poly(alkyleneoxy) substituent group according to formula (I), wherein each $R^1$ and each $R^2$ is independently H or $CH_3$, and each n is independently an integer of from about 5 to about 100.

**8.** The polymer of claim 1, wherein each of the monomeric units that comprise at least one pendant poly(alkyleneoxy) substituent group according to formula (I) that is derived from a monomer according to formula (II) is a monomeric unit according to formula (III):

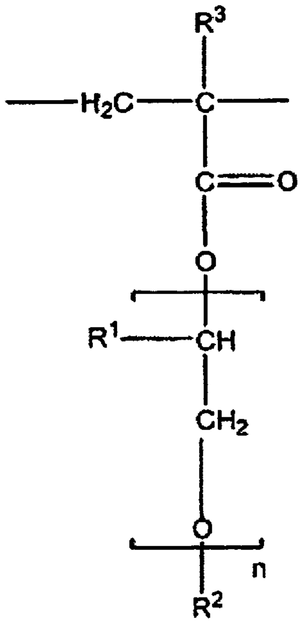

(III)

wherein:

each $R^1$ is independently H, methyl, or ethyl,
each $R^2$ is independently H or $(C_1-C_6)$alkyl,
each $R^3$ is independently H or $(C_1-C_4)$alkyl, and
each n is independently an integer of from about 5 to about 500.

**9.** The polymer of claim 8, wherein each $R^1$, each $R^2$, and each $R^3$ is independently H or $CH_3$, and each n is independently an integer of from about 5 to about 100

**10.** A personal care composition, comprising the polymer of claim 1 and a cosmetically acceptable carrier.

**11.** The personal care composition of claim 10, wherein the cosmetically acceptable carrier is selected from water, organic solvents, mixtures of two or more water miscible organic solvents, and mixtures of water and one or more water miscible organic solvents.

**12.** The personal care composition of claim 11, wherein the personal care composition is hair styling composition that comprises, based on 100 parts by weight of the personal care composition, from about 0.1 to about 10 pbw of the polymer and from about 90 to about 99.9 pbw of a carrier that comprises water.

**13.** The personal care composition of claim 11, wherein the personal care composition is a cleansing composition, the carrier comprises water, and the personal care composition further comprises one or more surfactants.

**14.** A method for treating hair or skin, comprising contacting the hair or skin with the polymer of claim 1.

## Patentansprüche

**1.** Polymer, umfassend beruhend auf der Gesamtanzahl von Monomereinheiten des Polymers mehr als 50 Prozent Monomereinheiten, die pro Monomereinheit mindestens eine angehängte Poly(alkyleneoxy)-Substituent-Gruppe gemäß der Formel (I) umfassen:

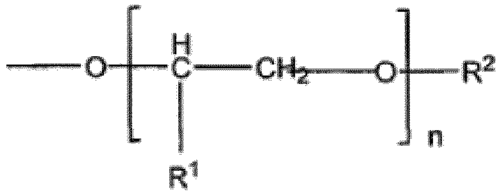

(I)

wobei:

jedes $R^1$ unabhängig H, Methyl oder Ethyl ist,
jedes $R^2$ unabhängig H oder ($C_1$-$C_6$)-Alkyl ist, und
jedes n unabhängig eine Ganzzahl von ca. 5 bis ca. 500 ist, und ein gewichtsgemitteltes Molekulargewicht von ca. 1.000 bis ca. 1.000.000 Gramm pro Mol aufweist,

und wobei jede der Monomereinheiten, die mindestens eine angehängte Poly(alkyleneoxy)-Substituent-Gruppe gemäß der Formel (I) umfasst, von einem Monomer gemäß der Formel (II) abgeleitet ist:

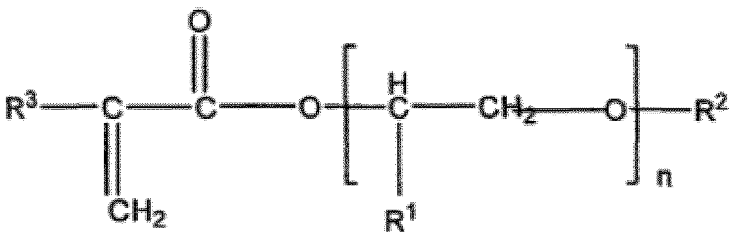

(II)

wobei:

jedes $R^1$ unabhängig H, Methyl oder Ethyl ist,
jedes $R^2$ unabhängig H oder ($C_1$-$C_6$)-Alkyl ist,
jedes $R^3$ unabhängig H oder ($C_1$-$C_4$)-Alkyl ist, und
jedes n unabhängig eine Ganzzahl von ca. 5 bis ca. 500 ist.

**2.** Polymer nach Anspruch 1, wobei das Polymer beruhend auf der Gesamtanzahl von Monomereinheiten des Polymers mehr als oder gleich wie ca. 80 Prozent Monomereinheiten umfasst, die pro Monomereinheit mindestens eine angehängte Poly(alkyleneoxy)-Substituent-Gruppe gemäß der Formel (I) umfassen.

**3.** Polymer nach Anspruch 1, wobei das Polymer ein Homopolymer ist.

**4.** Polymer nach Anspruch 1, wobei das Polymer beruhend auf der Gesamtanzahl von Monomereinheiten des Polymers Folgendes umfasst:

(a) von ca. 90 % bis weniger als 100 % erste Monomereinheiten, wovon jede pro Monomereinheit mindestens eine angehängte Poly(alkyleneoxy)-Substituent-Gruppe gemäß der Formel (I) enthält, und
(b) von mehr als 0 % bis ca. 10 zweite Monomereinheiten, die keine Poly(alkyleneoxy)-Substituent-Gruppen gemäß der Formel (I) enthalten.

**5.** Polymer nach Anspruch 1, wobei das Polymer ein gewichtsgemitteltes Molekulargewicht von ca. 2.500 bis ca. 900.000 Gramm pro Mol aufweist.

**6.** Polymer nach Anspruch 1, wobei das Polymer ein anzahlgemitteltes Molekulargewicht von ca. 2.500 bis ca. 750.000 Gramm pro Mol aufweist.

**7.** Polymer nach Anspruch 1, wobei jede der Monomereinheiten des Polymers mindestens eine angehängte Poly(alkyleneoxy)-Substituent-Gruppe gemäß der Formel (I) umfasst, wobei jedes $R^1$ und jedes $R^2$ unabhängig H oder $CH_3$ ist, und jedes n unabhängig eine Ganzzahl von ca. 5 bis ca. 100 ist.

**8.** Polymer nach Anspruch 1, wobei jede der Monomereinheiten, die mindestens eine angehängte Poly(alkyleneoxy)-Substituent-Gruppe gemäß der Formel (I), die von einem Monomer gemäß der Formel (II) abgeleitet ist umfassen, eine Monomereinheit gemäß der Formel (III) ist:

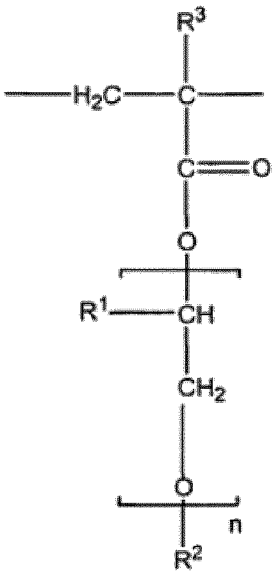

(III)

wobei:

jedes $R^1$ unabhängig H, Methyl oder Ethyl ist,
jedes $R^2$ unabhängig H oder $(C_1-C_6)$-Alkyl ist,
jedes $R^3$ unabhängig H oder $(C_1-C_4)$-Alkyl ist, und
jedes n unabhängig eine Ganzzahl von ca. 5 bis ca. 500 ist.

**9.** Polymer nach Anspruch 8, wobei jedes $R^1$, jedes $R^2$ und jedes $R^3$ unabhängig H oder $CH_3$ ist, und jedes n unabhängig an Ganzzahl von ca. 5 bis ca. 100 ist.

**10.** Körperpflegezusammensetzung, umfassend das Polymer nach Anspruch 1 und einen kosmetisch annehmbaren Träger.

**11.** Körperpflegezusammensetzung nach Anspruch 10, wobei der kosmetisch annehmbare Träger ausgewählt ist von Wasser, organischen Lösungsmitteln, Gemischen aus zwei oder mehrere wassermischbaren organischen Lösungsmitteln und Gemischen aus Wasser und einem oder mehreren wassermischbaren organischen Lösungsmitteln.

**12.** Körperpflegezusammensetzung nach Anspruch 11, wobei die Körperpflegezusammensetzung eine Haarmode-Zusammensetzung ist, die beruhend auf 100 Gewichtsanteilen der Körperpflegezusammensetzung von ca. 0,1 bis ca. 10 Gewichtsanteilen des Polymers und von ca. 90 bis ca. 99,9 Gewichtsanteilen eines Trägers, der Wasser umfasst, umfasst.

**13.** Körperpflegezusammensetzung nach Anspruch 11, wobei die Körperpflegezusammensetzung eine reinigende Zusammensetzung ist, der Träger Wasser umfasst und die Körperpflegezusammensetzung ferner ein oder mehrere Netzmittel umfasst.

**14.** Verfahren zum Behandeln von Haar oder Haut, umfassend ein Inkontaktbringen des Haars oder Haut mit dem Polymer nach Anspruch 1.

**Revendications**

**1.** Polymère comprenant, sur la base du nombre total d'unités monomériques du polymère, plus de 50 pour cent d'unités monomériques qui comprennent, par unité monomérique, au moins un groupe substituant de poly(alkylèneoxy) pendant selon la formule (I) :

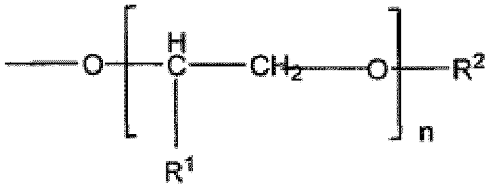

(I)

dans laquelle :

chaque $R^1$ représente indépendamment un atome H, un groupe méthyle ou éthyle,
chaque $R^2$ représente indépendamment un atome H ou un groupe alkyle en $C_1$ à $C_6$, et
chaque n représente indépendamment un entier d'environ 5 à environ 500, et présentant un poids moléculaire moyen en poids d'environ 1000 à environ 1000000 grammes par mole,

et dans laquelle chacune des unités monomériques qui comprennent au moins un groupe substituant de poly(alkylèneoxy) pendant selon la formule (I) provient d'un monomère selon la formule (II) :

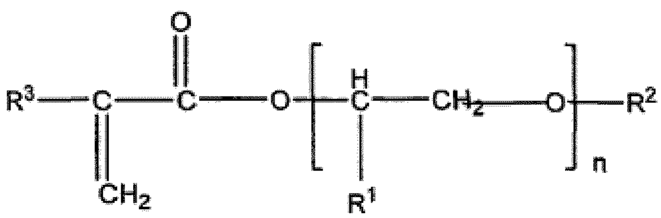

(II)

dans laquelle :

chaque $R^1$ représente indépendamment un atome H, un groupe méthyle ou éthyle,
chaque $R^2$ représente indépendamment un atome H ou un groupe alkyle en $C_1$ à $C_6$,
chaque $R^3$ représente indépendamment un atome H ou un groupe alkyle en $C_1$ à $C_4$, et

chaque n représente indépendamment un entier d'environ 5 à environ 500.

2. Polymère selon la revendication 1, ledit polymère comprenant, sur la base du nombre total d'unités monomériques du polymère, environ 80 pour cent d'unités monomériques ou plus qui comprennent, par unité monomérique, au moins un groupe substituant de poly(alkylèneoxy) pendant selon la formule (I).

3. Polymère selon la revendication 1, ledit polymère étant un homopolymère.

4. Polymère selon la revendication 1, ledit polymère comprenant, sur la base du nombre total d'unités monomériques du polymère :

(a) d'environ 90 % à moins de 100 % de premières unités monomériques, chacune desquelles comprenant, par unité monomérique, au moins un groupe substituant de poly(alkylèneoxy) pendant selon la formule (I), et
(b) de plus de 0 % à environ 10 secondes unités monomériques qui ne comprennent pas de groupes substituants de poly(alkylèneoxy) selon la formule (I).

5. Polymère selon la revendication 1, ledit polymère présentant un poids moléculaire moyen en poids d'environ 2500 à environ 900000 grammes par mole.

6. Polymère selon la revendication 1, ledit polymère présentant un poids moléculaire moyen en nombre d'environ 2500 à environ 750000 grammes par mole.

7. Polymère selon la revendication 1, chacune des unités monomériques du polymère comprenant au moins un groupe substituant de poly(alkylèneoxy) pendant selon la formule (I), chaque $R^1$ et chaque $R^2$ représentant indépendamment un atome H ou un groupe $CH_3$, et chaque n représentant indépendamment un entier d'environ 5 à environ 100.

8. Polymère selon la revendication 1, chacune des unités monomériques qui comprennent au moins un groupe substituant de poly(alkylèneoxy) pendant selon la formule (I) qui provient d'un monomère selon la formule (II) étant une unité monomérique selon la formule (III) :

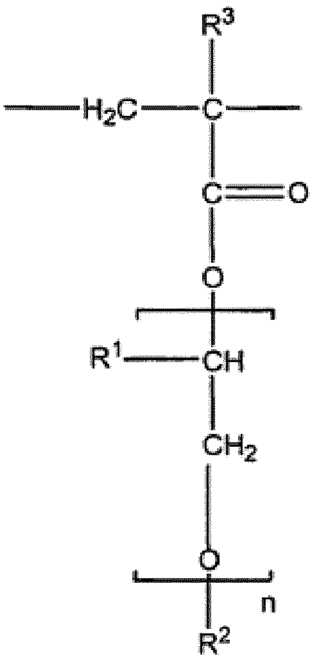

(III)

dans laquelle :

chaque $R^1$ représente indépendamment un atome H, un groupe méthyle ou éthyle,
chaque $R^2$ représente indépendamment un atome H ou un groupe alkyle en $C_1$ à $C_6$,
chaque $R^3$ représente indépendamment un atome H ou un groupe alkyle en $C_1$ à $C_4$, et

chaque n représente indépendamment un entier d'environ 5 à environ 500.

**9.** Polymère selon la revendication 8, chaque $R^1$, chaque $R^2$ et chaque $R^3$ représentant indépendamment un atome H ou un groupe $CH_3$, et chaque n représente indépendamment un entier d'environ 5 à environ 100.

**10.** Composition de soins d'hygiène personnelle, comprenant le polymère selon la revendication 1 et un excipient cosmétiquement acceptable.

**11.** Composition de soins d'hygiène personnelle selon la revendication 10, ledit excipient cosmétiquement acceptable étant choisi parmi l'eau, les solvants organiques, les mélanges de deux solvants organiques miscibles dans l'eau ou plus, et les mélanges d'eau et d'un ou plusieurs solvants organiques miscibles dans l'eau.

**12.** Composition de soins d'hygiène personnelle selon la revendication 11, ladite composition de soins d'hygiène personnelle étant une composition de coiffage qui comprend, sur la base de 100 parties en poids de la composition de soins d'hygiène personnelle, d'environ 0,1 à environ 10 parties en poids du polymère et d'environ 90 à environ 99,9 parties en poids d'un excipient qui comprend de l'eau.

**13.** Composition de soins d'hygiène personnelle selon la revendication 11, ladite composition de soins d'hygiène personnelle étant une composition nettoyante, l'excipient comprenant de l'eau et la composition de soins d'hygiène personnelle comprenant en outre un ou plusieurs tensioactifs.

**14.** Procédé de traitement des cheveux ou de la peau, comprenant la mise en contact des cheveux ou de la peau avec le polymère selon la revendication 1.